# EUROPEAN PATENT SPECIFICATION

(11) **EP 3 981 385 B1**
(45) Date of publication and mention of the grant of the patent: **21.06.2023**
(21) Application number: 20306171.8
(22) Date of filing: 08.10.2020
(51) Int. Cl.: A61K 8/37, A61Q 1/14, A61K 8/39, A61Q 19/00, A61Q 19/10

(54) **EMOLLIENT AND CLEANSING AGENT**
WEICHMACHENDES UND REINIGENDES MITTEL
ÉMOLLIENT ET AGENT DE NETTOYAGE

(43) Date of publication of application: 13.04.2022
(73) Proprietor: Oleon N.V., 9940 Ertvelde (BE)
(72) Inventor: FIEVEZ, Aurélie, 60600 CLERMONT (FR); PIERRE, Marjorie, 60280 CLAIROIX (FR); VAN DER WEEËN, Pieter, 9000 GENT (BE)
(74) Representative: Santarelli

(56) References cited:
- CN-A- 110 559 245
- DATABASE GNPD [Online] MINTEL; 12 September 2019 (2019-09-12), anonymous: "Cleansing Tissue in Water", XP055728039, Database accession no. 6864227

## Description

The present invention relates to a combination of two particular compounds and its use as emollient in cosmetics, in particular in cleansing compositions.

Most of cleansing compositions are water-based compositions comprising surfactants, in particular anionic surfactants for their good cleansing and foaming properties.

Sulfated surfactants such as sodium lauryl sulfate (SLS) or sodium lauryl ether sulfate (SLES) are anionic surfactants largely used in cosmetics for their very good cleansing and foaming properties, and their very low price.

However, those anionic surfactants and in particular sulfated surfactants, can cause skin irritation. The cosmetic industry, in order to balance this side effect, developed more and more hygiene and skin care products with hydration property, thus, in most of the case, by introducing an emollient.

An emollient increases the hydration, or the moisture content of the skin, by reducing evaporation of water from the skin and soothing irritation and providing a soft and smooth feel to the skin.

In general, emollients are hydrophobic, such as vegetable oils, silicones and mineral oils.

Since water is often an important component of many formulations, hydrophobic emollients and water are usually combined under the form of an emulsion (water in oil, oil in water or multiple emulsions) where mostly an emulsifier, i.e. a surfactant having emulsifying property, is added to facilitate the emulsification and increase the thermodynamic stability of the emulsion. The emulsion being white opaque, a solubilizer is then added to restore the transparency. But the use of solubilizers have several drawbacks. Solubilizers are not universal solutions, i.e. their use is usually suitable for one specific emulsion system that has to be designed depending on the product to solubilize. Moreover, to obtain a complete transparency, an high percentage of solubilizer is often needed which induces a price increase, and may have an impact on the final cosmetic composition's organoleptic properties, foam stability and/or viscosity.

To avoid using an additional surfactant and solubilizer, and maintain transparency of the cosmetic composition, hydrophilic emollients appears then as a better solution. Several molecules already exist onto the market, mainly polyethylene glycol (PEG) derivatives, such as PEG-7 glyceryl cocoate, PEG-6 caprylic/capric glycerides.

However, there are some human health concerns about use of PEG derivatives. CN 110 559 245 A (WUHAN BEBEVISA BIOTECH CO LTD) 13 December 2019 discloses a composition comprising polyglyceryl-4 caprate and glyceryl caprylate.

Therefore, there is still a need to an hydrophilic emollient that would present one or more of the following characteristics:
- totally soluble or dispersible in water;
- preserving transparency of the cosmetic composition;
- presenting hydration property, even in presence of anionic surfactants.

The Applicant surprisingly found that a specific combination presents not only all those characteristics but also have additional advantages such as cleansing property.

Accordingly, the present invention relates to a combination comprising or consisting of polyglyceryl-4 caprate and glyceryl monocaprylate, wherein the weight ratio polyglyceryl-4 caprate / glyceryl monocaprylate is comprised between 45/55 and 55/45.

In the present application, unless otherwise indicated, all ranges of values used are to be understood as being inclusive limits.

Polyglyceryl-4 caprate or polyglycerol-4 caprate (PG-4 caprate) commonly results from the esterification reaction between capric acid and polyglycerol-4.

The polyglycerol-4 derives itself from the homopolymerization reaction of glycerol. This reaction of homopolymerization of glycerol consists in reacting together several molecules of glycerol via their hydroxyl functions resulting in the formation of a molecule containing several glycerol units linked by ether bonds.

The integer following "polyglyceryl" or "polyglycerol" represents the number of glycerol units forming the polyglycerol.

However, during the reaction of homopolymerization, a mixture of polyglycerols with different number of glycerol units are usually formed. The integer represents then the average of the number of glycerol units determined by calculating the weight percentage of the different polyglycerols present in the mixture.

Thus, the polyglyceryl-4 caprate results preferably from esterification reaction between capric acid and a mixture of polyglycerols or glycerol oligomers, wherein the average of the number of glycerol units based on the weight percentage of different polyglycerols is of 4.

More particularly, the polyglyceryl-4 caprate results from esterification reaction between capric acid and a mixture of glycerol oligomers, comprising 30-45 wt% of triglycerol, 25-45 wt% of tetraglycerol, 15-35 wt% of pentaglycerol and hexaglycerol, less than 10 wt% of diglycerol and less than 15 wt% of heptaglycerol and higher oligomers.

Glyceryl monocaprylate or glyceryl caprylate (CAS n°26402-26-6), is known as emollient.

However, the combination according to the invention present an improved emollient property and additional technical effects, such as cleansing property as illustrated by Example 4.

The combination according to the invention is advantageously a colorless transparent liquid at 25°C and atmospheric pressure.

Advantageously, the combination according to the invention is a renewable product. Indeed, both of its components can be produced from vegetable oils.

Preferably, the weight ratio polyglyceryl-4 caprate / glyceryl monocaprylate is of 50/50.

The present invention also relates to a process for preparing a combination by mixing a polyglyceryl-4 caprate and a glyceryl monocaprylate, wherein the weight ratio polyglyceryl-4 caprate / glyceryl monocaprylate is comprised between 45/55 and 55/45.

The combination according to the invention and its compounds are as described above, including preferential and advantageous features.

Preferably, the mixing is performed at a temperature of at least 30°C, more preferably of at least 40°C, even more preferably of at least 50°C.

Preferably, the weight ratio polyglyceryl-4 caprate / glyceryl monocaprylate is of 50/50.

The present invention also concerns the use of the combination according to the invention as an emollient.

The combination according to the invention presents hydration property, in particular, a hydration property that lasts in time.

As evidenced by Examples 3, 5 and 6, in presence of surfactants with cleansing property, the combination according to the invention allows to maintain water in the skin (positive electrical conductivities could be measured), even with surfactant such as SLES (particularly irritant for the skin).

The present invention also concerns the use of the combination according to the invention, as a cleansing agent.

As illustrated by Example 4, the combination according to the invention exhibits better cleansing property than SLES. The combination according to the invention can clean up make-up, even resistant ones.

Advantageously, the combination according to the invention is used as emollient and as cleansing agent.

As evidenced by Examples 3-6, the combination according to the invention exhibits both hydration property and cleansing property.

The present invention also concerns a composition comprising :
- polyglyceryl-4 caprate ;
- glyceryl monocaprylate ; and
- water;
wherein the weight ratio polyglyceryl-4 caprate / glyceryl monocaprylate is comprised between 45/55 and 55/45.

The polyglyceryl-4 caprate and glyceryl monocaprylate are as described above, including preferential and advantageous features.

The composition according to the invention is advantageously a transparent liquid at 25°C and atmospheric pressure.

The total quantity of polyglyceryl-4 caprate and glyceryl monocaprylate, in the composition according to the invention, is preferably of at least of 1wt%, more preferably at least 2 wt% based on the weight of the composition.

By "total quantity of polyglyceryl-4 caprate and glyceryl monocaprylate", it is intended the quantity of all molecules of polyglyceryl-4 caprate and glyceryl monocaprylate.

The total quantity of polyglyceryl-4 caprate and glyceryl monocaprylate, in the composition according to the invention, is preferably of at most 20 wt%, more preferably at most 10 wt%, even more preferably at most 5 wt% based on the weight of the composition.

Preferably, the total quantity of polyglyceryl-4 caprate and glyceryl monocaprylate in the composition according to the invention, is comprised between 1 and 10 wt%, more preferably between 1 and 5 wt%, even more preferably between 2 and 5 wt% based on the weight of the composition.

Advantageously, the composition according to the invention presents an hydrolytic stability. Subjected to different pH from 2.8 to 11.3, the acid value stays constant (no more than 0.5 mg KOH/g after 30 days at 25°C).

The acid values were measured according to standard AOCS Cd 3d-63(03).

Advantageously, the composition according to the invention presents anti-microbial property.

Preferably, the weight ratio polyglyceryl-4 caprate / glyceryl monocaprylate is of 50/50.

Advantageously, the composition according to the invention, further comprises a surfactant, a thickening agent and/or a preservative.

The preservative may be chosen among the group consisting of sodium benzoate, sorbic acid, glycols, phenoxyethanol, parabens, methylchloroizothiazolinone, methylisothiazolinone or ethylhexylglycerin.

The thickening agent may be chosen among the group consisting of cocamide DEA, sodium chloride, acrylates crosspolymers, gums such as xanthan, carraghenan or polysaccharides such as cellulose and starch.

The surfactant may have emulsifying property or cleansing property and optionally foaming property.

In a first embodiment, the composition according to the invention comprises a surfactant other than a surfactant having cleansing property and optionally foaming property.

As illustrated in Example 4, the combination according to the invention exhibits a better cleaning property than SLES, and can be used as a cleaning agent.

In a second embodiment, the composition according to the invention comprises a surfactant having cleansing property and optionally foaming property.

The surfactant having cleansing property and optionally foaming property is preferably used in the field of cosmetics.

Usually, a surfactant having cleansing property and optionally foaming property is an anionic or an amphoteric surfactant.

Examples of surfactant having cleansing property are, cocamidopropyl-betaine, decyl glucoside and cetyl betaine.

Examples of surfactant having cleansing and foaming properties are sodium lauryl ether sulfate, sodium lauryl sulfate, ammonium lauryl sulfate, disodium-laureth-sulfosuccinate, sodium-lauryl-sulfoacetate, coco-glucoside, lauryl-glucoside, sodium-cocoamphoacetate, arcosinates, taurates.

The combination according to the invention is soluble in compositions comprising a surfactant, in particular an anionic or an amphoteric surfactant.

Those compositions according to the invention are transparent.

Advantageously, the combination according to the invention doesn't destabilize the foam that can be formed by the presence of a surfactant having foaming property in the composition according to the invention.

In Examples 3-6, it is shown that when the composition according to the invention comprises a combination according to the invention and a surfactant having cleansing property and optionally foaming property, the hydration of the skin is better and the cleansing of make-up is better than compositions comprising a surfactant having cleansing property and optionally foaming property and free of combination according to the invention.

Moreover, there is a boosting effect on the cleansing property for particular weight ratio (surfactants having cleansing property and optionally foaming property) / (combination according to claim 1 ).

Advantageously, in the composition according to the invention, the surfactant is a surfactant having cleansing property and optionally foaming property and wherein the weight ratio (surfactants having cleansing property and optionally foaming property) / (combination according to claim 1) is comprised between 4 and 3.

In a particular embodiment of the second embodiment, the surfactant with cleansing property and optionally foaming property is a sulfated surfactant.

The sulfated surfactant is preferably sodium lauryl ether sulfate, sodium lauryl sulfate, ammonium lauryl sulfate.

There is a boosting effect on cleaning property when combination according to the invention and sulfated surfactant are used in a same composition. This effect is more visible when the make-up is resistant, as illustrated in Example 4.

Preferably, the weight ratio (sulfated surfactant) / (combination according to the invention) is of 4.

In a preferred embodiment of the second embodiment, the surfactant having cleansing property and optionally foaming property is not a sulfated surfactant.

In other words, the composition according to the invention is free of sulfated surfactant.

The surfactant with cleansing property and optionally foaming property which is not a sulfated surfactant may be a disodium-laureth-sulfosuccinate, a sodium-lauryl-sulfoacetate, a coco-glucoside, a sodium-cocoamphoacetate, an arcosinate, a taurates, a lauryl-glucoside, a cocamidopropyl-betaine, a decyl glucoside, a cetyl betaine, or mixture thereof.

Preferably, the surfactant with cleansing property and optionally foaming property which is not a sulfated surfactant is chosen among the group consisting of a coco-glucoside, a sodium-cocoamphoacetate, a lauryl-glucoside, a cocamidopropyl-betaine, a decyl glucoside, a cetyl betaine, or mixture thereof.

More preferably, the surfactant with cleansing property and optionally foaming property which is not a sulfated surfactant is chosen among the group consisting of a coco-glucoside, a lauryl-glucoside, a sodium-cocoamphoacetate or mixture thereof.

The present invention also concerns the preparation of the composition according to the invention, by mixing into water, polyglyceryl-4 caprate and glyceryl monocaprylate, and optionally a surfactant, a thickening agent and/or a preservative.

According to a first embodiment, polyglyceryl-4 caprate and glyceryl monocaprylate are added together into water. Preferably a combination of polyglyceryl-4 caprate and glyceryl monocaprylate according to the invention is prepared prior to contact with water.

According to a second embodiment, polyglyceryl-4 caprate and glyceryl monocaprylate are added into water separately.

The present invention also relates to the use of the composition according to the invention as a cosmetic composition.

The cosmetic composition can be in the form of a liquid having a large viscosity range, a water-in-oil emulsion, a gel.

The cosmetic composition can further comprise additional ingredients that are typically used in cosmetics, such as a hair conditioning agent, a cosmetic active ingredient and/or a pigment or a colorant.

The cosmetic composition can be a cleansing composition, a cream, a hair conditioner.

Preferably, the cosmetic composition is a cleansing composition.

Thus, the present invention also relates to a cleansing composition free of sulfated surfactant comprising a polyglyceryl-4 caprate, a glyceryl monocaprylate and water, wherein the weight ratio polyglyceryl-4 caprate / glyceryl monocaprylate is comprised between 45/55 and 55/45.

The cleansing composition, is preferably a shampoo, a shower gel, a micellar water, a make-up remover or a face cleanser.

The present invention concerns a method to lower the quantity of sulfated surfactant having cleansing property and optionally foaming property, in a cleansing composition, by adding a combination according to claim 1 into said cleansing composition.

The combination according to the invention and the sulfated surfactant are as described above, including preferential and advantageous features.

Preferably, the quantity of combination is of at least 1 wt%, more preferably at least 2 wt% based on the weight of the cleansing composition.

Preferably, the quantity of sulfated surfactant having cleansing property and optionally foaming property is lowered by at least 20 wt%, more preferably by at least 30 wt%.

Preferably, the weight ratio (sulfated surfactant having cleansing property and optionally foaming property) / (combination according to the invention) is comprised between 4 and 3, more preferably is of 4.

The invention is further described in the following examples. It will be appreciated that the invention as claimed is not intended to be limited in any way by these examples.

### Chemicals used in Examples

- Polyglyceryl-4 caprate (PG-4 caprate, Radia 7932 from Oleon);
- Glyceryl monocaprylate (Jolee 7907 from Oleon);
- Demineralized water (aqua);
- Surfactants with cleansing property and optionally foaming property:
   - sodium lauryl ether sulfate (SLES) 70 wt% active matter, CAS n° 68585-34-2;
   - Cocamidopropyl betaine 30 wt% active matter, Dehyton K from BASF;
   - Sodium cocoamphoacetate, Dehyton MC from BASF;
   - Coco glucoside, Plantacare 818 UP from BASF;
   - Lauryl glucoside, Plantacare 1200 UP from BASF;
- Sodium chloride (NaCl);
- Cocamide DEA, Empilan 2302 from Sigma;
- Sodium benzoate, Purox S from Emerald Kalama Chemical
- Make-up:
   - waterproof mascara : Lash Sensational from Maybelline;
   - lipstick Superstay Matte Ink from Maybelline.

### Example 1: Preparation of combinations

### 1.1 Combinations 1-3 according to the invention

Combinations 1 to 3 were prepared by mixing in a flask polyglyceryl-4 caprate and glyceryl monocaprylate in respective weight ratio polyglyceryl-4 caprate/glyceryl monocaprylate of 50/50, 45/55 and 55/45. The combinations were heated at 60°C and mixed under gentle stirring until complete homogenization.

The combinations were then cooled down to room temperature.

With weight ratio polyglyceryl-4 caprate/glyceryl monocaprylate comprised between 45/55 and 55/45, colorless transparent liquids, with a kinematic viscosity similar to the one of water at 25°C, were obtained.

### 1.2 Comparative combinations 1-6

Comparative combinations 1-6 were prepared by mixing polyglyceryl-4 caprate and glyceryl monocaprylate according to the method described in Example 1.1 with the following respective weight ratio polyglyceryl-4 caprate/glyceryl monocaprylate of 90/10, 75/25, 60/40, 40/60, 25/75 and 10/90.

Aspects of those comparative combinations are described in Table 1 below.

**Table 1: Aspect of comparative combinations 1-6**

| Comparative combination | Weight ratio PG-4 caprate / glyceryl monocaprylate | Aspect |
|---|---|---|
| 1 | 90/10 | non-transparent solid |
| 2 | 75/25 | non-transparent viscous liquid |
| 3 | 60/40 | non transparent pourable liquid |
| 4 | 40/60 | yellowish non-transparent solid |
| 5 | 25/75 | yellowish non-transparent solid |
| 6 | 10/90 | yellowish non-transparent solid |

None of these ratio allowed to obtain a transparent liquid with a kinematic viscosity similar to the one of water.

### Example 2: Preparation of compositions

### 2.1 Composition 1 according to the invention

Composition 1 was prepared by solubilizing 5 g of combination 1 in 95 g of demineralized water.

The composition 1 obtained was a colorless and transparent liquid.

### 2.2 Composition 2 according to the invention

Composition This was prepared by solubilizing 2.5 g of polyglyceryl-4 caprate and 2.5 g of glyceryl monocaprylate in 95 g of demineralized water.

The composition 1bis obtained was a colorless and transparent liquid.

### 2.3 Composition 3 according to the invention

Composition 3 was prepared by solubilizing 1 g of combination 1 and 5.7 g of SLES 70 wt%, (i.e. 4 g of SLES), in 93.3 g of demineralized water.

The composition 2 obtained was a colorless and transparent liquid.

### 2.4 Comparative composition 1-5

Comparative compositions 1-2 were prepared by solubilizing respectively 5 g of PG-4 caprate and 5 g of glyceryl monocaprylate in 95 g of demineralized water.

Comparative composition 3 was prepared by solubilizing 5.7 g of SLES 70 wt% (i.e. 4 g of SLES), in 94.3 g of demineralized water.

Comparative compositions 4-5 were prepared by solubilizing 5.7 g of SLES 70 wt% (i.e. 4 g of SLES), and respectively 1 g of PG-4 caprate and 1 g of glyceryl monocaprylate, in 93.3 g of demineralized water.

### Example 3: Hydration property

To evaluate the hydration property, the electrical conductivity, or capacitance, of the skin was measured, which is directly influenced by its water content. Indeed, the more the skin is moisturized, the higher the electrical conductivity value.

### 3.1 Material

- a probe Corneometer CM825 from Courage and Khazaka;
- Composition 3 according to the invention;
- Comparative compositions 3-5.

### 3.2 Method

Three days prior to the measurement, 3 people began washing their forearms with a neutral soap bar. The use of all personal care products (e.g., lotions, creams) on their forearms were then prohibited until the measurement.

Tests were performed at a place with fixed temperature (T= 20°C) and humidity (52-53%), in a quiet environment, where the people rested 30 minutes before performing the test.

The surface of the forearms were gently wiped with a damp disposable washcloth and patted dry with a paper towel.

8 circles were marked on forearms (4 on left side for zones to treat and 4 on right side, non-treated zone). On each center of the 4 circles on left side, were applied 25 µL of a composition to test. To apply the compositions on the complete circle spot, 10 rubs with a finger were made.

After 1 minute rest, 25µL of water were applied onto the center of each 8 circles, following by rubs as described previously.

After an additional minute of rest, a cotton was applied on each circle and pressed during 10 seconds to remove the excess.

Then, the corneometer probe was placed onto each circle without any pressure to evaluate the electrical conductivity of the skin immediately after the washing and every 30 minutes for 2 hours. Each time, 5 values were taken and the average was then used.

For each person, the difference in electrical conductivity between the average value of the treated spot (left side onto the skin) and the average value of the untreated spot (right side onto the skin) was determined every 30 minutes.

Results in Table 2 were obtained by averaging the measured electrical conductivities of the 3 people over a two-hour period.

To be considered as hydrating, the difference between the skin treated and non-treated should be above 4.

**Table 2: Electrical conductivity of the skin over time**

| | 0 h | 0.5 h | 1h | 1.5 h | 2h |
|---|---|---|---|---|---|
| Composition 3 | 78.5 mS | 25.3 mS | 6.5 mS | 5.5 mS | 5.5 mS |
| Comparative composition 3 | 47.6 mS | 0 mS | 0.2 mS | 0 mS | 0.3 mS |
| Comparative composition 4 | 32.2 mS | 4.4 mS | 1.1 mS | 0.5 mS | 0.2 mS |
| Comparative composition 5 | 30.1 mS | 5.8 mS | 1.0 mS | 0.5 mS | 0.2 mS |

It can be observed that immediately after the application of a composition on the skin, the later present a certain electrical conductivity due to the application of water on the skin just before the measurement. But after 30 minutes, it can be seen that water evaporates from skin more or less depending on the use or not of a combination according to the invention. While the electrical conductivity of the skin of the forearm after treatment with comparative compositions are at 1.1 mS or lower after 1 hour, the electrical conductivity after treatment of the skin with the composition 3 according to the invention is of 6.5mS after 1 hour and still of 5.5 mS after 2 hours.

As expected, washing the skin with water comprising SLES doesn't retain water in the skin. Addition of PG-4 caprate or monoglyceryl caprylate improves the hydration of the skin during the first 30 minutes following the washing. But adding the combination according to the invention into the water comprising SLES, allows not only a much higher hydration of the skin 30 minutes after the washing, but also the lasting of the hydration of the skin up to at least 2 hours.

### Example 4: Cleansing property

In order to test this property, a method based on colorimetry was used.

### 4.1 Material

- Skin colorimeter CL 400 from Courage and Khazaka;
- Transparent film roll : Fixomull from BSN medical;
- Make-up:
   - waterproof mascara : Lash Sensational from Maybelline;
   - lipstick: Superstay Matte Ink from Maybelline;
- Compositions tested:
   - Composition 1 and 3 according to the invention
   - Comparative compositions 1-5.

### 4.2 Method

On a white support, two transparent film rolls were fixed.

The L* value was measured 4 times with the skin colorimeter.

L* value comes from CIE L*a*b* or CIELAB color space. L* expresses the lightness from black (0) to white (100).

The mascara and the lipstick were applied with the same force in pre-defined circles of 1.5 cm diameter. To apply the make-up on the complete circle spots, 10 rubs with a finger were made.

After a drying time of 10 minutes at 40°C, the L* value of each circle was measured 4 times.

Then each circle spot was cleaned by rubbing 10 times the make-up spot with a cotton soaked with 2 mL of the composition tested.

After a drying time of 10 minutes at 40°C, the L* value of each circle was measured 4 times and the average was then used.

In Tables 3 and 4 are gathered ΔL*values, i.e. the difference between average L* value obtained after cleansing and the average L* value obtained with the make up before cleansing (average L* value after cleansing - average L* value of the make-up).

The higher ΔL* value is, the better the efficiency of cleansing.

**Table 3: ΔL*values after cleansing of mascara**

| | ΔL* |
|---|---|
| Composition 1 | 36 |
| Comparative composition 1 | 25 |
| Comparative composition 2 | 26 |
| Comparative composition 3 | 26 |

The composition 1 cleans better the mascara than the comparative compositions 1 and 2 comprising respectively PG-4 caprate and monoglyceryl caprylate. The combination according to the invention cleans better the mascara than each of the two components of the combination according to the invention used alone, and even better than SLES (comparative composition 3).

Moreover, there is a synergistic effect between the components of the combination according to the invention. Indeed, the ΔL* value obtained with the combination according to theinvention is higher than the sum of ΔL* values obtained with each components used separately.

**Table 4: ΔL* values after cleansing of lipstick**

| | ΔL* |
|---|---|
| Composition 1 | 23 |
| Comparative composition 1 | 10 |
| Comparative composition 2 | 13 |
| Composition 3 | 36 |
| Comparative composition 3 | 18 |
| Comparative composition 4 | 19 |
| Comparative composition 5 | 13 |

The lipstick used in this example is more resistant to cleansing than the mascara, as it can be seen by comparing ΔL* values (26 with mascara and 18 with lipstick) of comparative composition 3 (comprising 4% active matter of SLES) in Table 3 and 4.

Once again, the combination 1 according to the invention cleans better the lipstick than the two components of the combination according to the invention used alone (comparative composition 1 and 2), and better than SLES (comparative composition 3).

Moreover, in presence of SLES, the combination according to the invention increases significantly the cleansing (ΔL* of 36 with composition 3) while the two components of the combination used alone with SLES have no impact on the cleansing (ΔL* of 13-19 with comparative compositions 3-5).

Indeed, when a weight ratio SLES/ combination according to the invention of 4/1 is used, the ΔL* value is higher than the sum of ΔL* values obtained with each components used separately. Thus, there is a synergistic effect between SLES and the combination according to the invention when used in a weight ratio SLES/ combination according to the invention of 4/1.

### Example 5: Lowering the quantity of sodium lauryl ether sulfate (SLES)

### 5.1 Decreasing the critical micelle concentration (CMC) of composition comprising sodium lauryl ether sulfate (SLES)

The critical micelle concentration (CMC) is defined as the minimum amount of surfactant required to obtain micelles in a liquid.

To determine the CMC, the Wilhelmy plate method were used with a tensiometer K100 from Kruss.

At 25°C, the combination 1 according to the invention was diluted at different concentrations in water (from 10 g/L to 0,00001 g/L). The surface tension of each of those compositions was measured. At high concentration (10g/L until 0,01g/L), foam could appear, then the surface tension was measured after 1 day of rest.

The same protocol was followed with the combination 1 and each of its components, but mixed with SLES in a weight ratio SLES/combination or component of 4/1.

Then, the curve surface tension = f(concentration) was plotted for each composition, and by a tangent evaluation, the CMC was determined and results are gathered in Table 5.

**Table 5: CMC in water**

| | CMC (ppm) |
|---|---|
| Composition 1 | 11 |
| Composition 3 | 114 |
| Comparative composition 3 | 1000 |
| Comparative composition 4 | 1000 |
| Comparative composition 5 | 980 |

It can be observed that the composition 1 according to the invention presents a low CMC.

By comparing CMC values of composition 3 and comparative composition 3, it can be said, that the addition of a combination according to the invention into a composition comprising SLES and water lowers the CMC of said composition. On the opposite, adding only one component of the combination according to the invention, doesn't lower the CMC of the SLES in water.

If the combination according to the invention can lower the CMC of a composition comprising SLES, it means that the quantity of SLES can be reduced to obtain a composition less irritant while maintaining the cleansing performance. Indeed, surfactants with cleansing property solubilize the poorly soluble hydrophobic material (such as oils) by forming micelles around them that can then be easily washed off with water.

### 5.2 Cleansing compositions with SLES

To illustrate the lowering of quantity of SLES by adding a combination according to the invention, three cleansing compositions according to the invention (CC 1-3) and one reference composition (R1) were prepared.

### 5.2.1 Preparation of cleansing compositions CC 1-3 and R 1

Chemicals and their quantities are described in Table 6 below.

To prepare those cleansing compositions, demineralized water (A) was first introduced into a beaker, agitated by a 4-blade propeller (500 rpm) and heated at 60°C.

B was incorporated slowly until complete dispersion.

Then the heating was stopped.

C was added, followed by D and E.

The pH was then controlled and adjusted if needed to 4,5-5 by adding citric acid or sodium hydroxide.

Cleansing compositions thus obtained were transparent.

**Table 6: Cleansing compositions 1-3 and reference cleansing composition R1**

| | Chemicals | Function | R 1 %W/W | CC 1 %W/W | CC 2 %W/W | CC 3 %W/W |
|---|---|---|---|---|---|---|
| A | Aqua | Solvent | Q.S | Q.S | Q.S | Q.S |
| B | SLES 70 % active matter | Cleansing, foaming | 21.42 (15% AM*) | 21.42 (15% AM*) | 12.85 (9% AM*) | 7.14 (5% AM*) |
| C | Cocamidopropyl betaine 30 % active matter | Cleansing, foam boosting | 10 (3% AM*) | 10 (3% AM*) | 10 (3% AM*) | 10 (3% AM*) |
| D | Combination 1 | Emollient | 0 | 3 | 3 | 5 |
| E | NaCl | Thickening agent | 0,7 | 0,7 | 0,7 | 0,7 |
| | Sodium benzoate | Preservative | 0,8 | 0,8 | 0,8 | 0,8 |

| | | | | | | |
|---|---|---|---|---|---|---|
| *"AM" stand for active matter and % are weight percentages based on weight of the composition | | | | | | |

### 5.2.2 Characteristics of cleansing compositions

All results are gathered in Table 7 below.

**Table 7: Characteristics of cleansing compositions 1-3 according to the invention and reference cleansing composition R1**

| | R 1 | CC 1 | CC 2 | CC 3 |
|---|---|---|---|---|
| Dynamic viscosity (mPa.s) | 2660 | 3630 | 2590 | water like |
| Volume of foam (mL) at t= 0min | 520 | 530 | 590 | 570 |
| Volume of foam (mL) at t= 10min | 500 | 510 | 540 | 520 |
| Volume of foam (mL) at t= 30min | 390 | 450 | 490 | 420 |
| Electrical conductivity (mS) at t= 0 | 38,4 | 38,4 | 39 | 29,4 |
| Electrical conductivity (mS) at t= 30 min | 4,2 | 9,6 | 8,6 | 10,4 |
| Electrical conductivity (mS) at t= 1 h | 1,6 | 9,4 | 10,8 | 10,4 |
| Electrical conductivity (mS) at t= 2 h | 0,4 | 5,6 | 6,4 | 7,8 |
| ΔL* with lipstick | 1,46 | 5,1 | 12,81 | 4,7 |

### a) Dynamic viscosity

Dynamic viscosity of each composition was measured using a viscosimeter Brookfield RDDV-E, spindle 3, 10 rpm, 1 min, at 25°C.

It can be observed by comparing viscosities of R 1 and CC 1, that addition of 3 wt% of a combination according to the invention into a composition comprising 15 w% (active matter) of SLES, increases slightly the viscosity of the resulting composition. If then the quantity of SLES is lowered (CC 2), then the viscosity is similar to the viscosity of the composition comprising more SLES and no combination according to the invention (R 1).

### b) Foam stability

Volume of foam was measured to evaluate the stability of foam.

Foam was created by passing air flow through a porous stone which was submerged in a composition to test. Airs bubbles thus created raised to the surface where a layer of foam was built up.

### Method

1 wt% of a cleansing composition was diluted in 99 wt% of tap water.

200 mL of the resulting composition were placed in a 1L tub, which was put in a thermostated bath at 38°C.

The air diffuser was placed into the test tube near the 100 mL graduation and air was introduced for 5 min at a flow rate of 94 mL/min.

The volume of foam was measured just after switch off the air diffusion and after 10, and 30 minutes.

It can be observed that the volume of foam is stable, it is not impacted by the presence of the combination in cleansing compositions, since volumes of foam of CC 1-3 are similar or slightly larger than volume of foam of R 1.

Moreover, results obtained with CC 2 demonstrates that it is possible to lower quantity of SLES without decreasing the volume of foam.

### c) Hydration property

The electrical conductivity was measured according to the method described in Example 3.2.

After 2 hours, the electrical conductivity is still positive after washing with the three cleansing compositions, and even slightly higher when the quantity of SLES decreases (the value obtained with CC 3 is slightly higher than the one of CC 2 which is also slightly higher than the one of CC 1).

### d) Cleansing property

L* values were measured according to the method described in Example 4.2 and ΔL* are given in Table 7.

By comparing R 1 and CC 1, it can be seen that adding 5wt% of a combination according to the invention to 18wt% of surfactants, weight percentage being based on the weight of the cleansing composition, the ΔL* value increases. In particular, the ΔL* value after 2 hours is still of 5.6 mS, while is closed to 0 without the combination according to the invention.

Moreover, there is a boosting effect of the combination according to the invention on the cleansing property, such as observed in Example 4 with the lipstick.

By reducing more the SLES quantity and increasing the quantity of the combination according to the invention, reaching a weight ratio surfactants / combination of 8/5 (CF 3), the foam volume obtained is still the same, and the hydration property and the cleansing property are similar to those observed with CC 1.

### Example 6: Cleansing compositions with surfactants other than sodium lauryl ether sulfate (SLES)

### 6.1 Preparation of cleansing compositions CC 4 and R 2

Preparation of those cleansing compositions follows method described in Example 5.2.2, using chemicals and quantities as described in Table 8 below.

Cleansing compositions thus obtained were transparent. The combination according to the invention is soluble in compositions comprising amphoteric surfactants such as glucosides.

**Table 8: Cleansing composition 4 according to the invention and reference cleansing composition R 2**

| | Chemicals | Function | R2 %W/W | CC4 %W/W |
|---|---|---|---|---|
| A | Aqua | Solvent | Q.S | Q.S |
| B | Sodium cocoamphoacetate | Cleansing, foaming | 6 | 6 |
| | Coco glucoside | Cleansing, foaming | 4 | 4 |
| | Lauryl glucoside | Cleansing, foaming | 2 | 2 |
| C | Combination 1 | Emollient | 0 | 3 |
| D | Cocamide DEA | Thickening agent | 3 | 3 |
| | Sodium benzoate | Preservative | 0,8 | 0,8 |

### 6.2 Characteristics of cleansing compositions

All results are gathered in Table 9 below.

Methods to measure the different characteristics are as described in Example 5.2.3.

**Table 9: Characteristics of cleansing composition 4 and reference cleansing composition R2**

| | R 2 | CC 4 |
|---|---|---|
| Viscosity | water like | water like |
| Volume of foam (mL) at t= 0min | 640 | 650 |
| Volume of foam (mL) at t= 10min | 650 | 650 |
| Volume of foam (mL) at t= 30min | 570 | 570 |
| Electrical conductivity (mS) at t= 0 | 88,4 | 82,4 |
| Electrical conductivity (mS) at t= 30min | 6,6 | 6,8 |
| Electrical conductivity (mS) at t= 1 h | 6,8 | 6,2 |
| Electrical conductivity (mS) at t= 2h | 4,6 | 6,4 |
| ΔL* with lipstick | 1,49 | 6,83 |

Addition of the combination according to the invention to a cleansing composition comprising amphoteric surfactants having cleansing properties, such as glucosides, doesn't modify the viscosity, nor destabilize the foam. Indeed, volumes of foam obtained with R 2 and CC 4 are similar immediately after their formation and after 30 minutes.

Cleansing composition 4 maintain the skin hydration with a slightly improvement after 2 hours compare to R 2 which do not comprise a combination according to the invention, as it is shown by the electrical conductivity values.

The cleansing property is improved by addition of a combination according to the invention to the glucoside surfactants, as shown by the greater ΔL* value for CC 4 compared to the one of R 2.

## Claims

1. Combination comprising or consisting of polyglyceryl-4 caprate and glyceryl monocaprylate, wherein the weight ratio polyglyceryl-4 caprate / glyceryl monocaprylate is comprised between 45/55 and 55/45.

2. Process for preparing a combination by mixing a polyglyceryl-4 caprate and a glyceryl monocaprylate, wherein the weight ratio polyglyceryl-4 caprate / glyceryl monocaprylate is comprised between 45/55 and 55/45.

3. Use of the combination according to claim 1 as an emollient.

4. Use of the combination according to claim 1, as a cleansing agent.

5. Composition comprising :
- polyglyceryl-4 caprate ;
- glyceryl monocaprylate ; and
- water;
wherein the weight ratio polyglyceryl-4 caprate / glyceryl monocaprylate is comprised between 45/55 and 55/45.

6. Composition according to claim 5, further comprising a surfactant, a thickening agent and/or a preservative.

7. Composition according to claim 6, wherein the surfactant is a surfactant having cleansing property and optionally foaming property and wherein the weight ratio (surfactants having cleansing property and optionally foaming property) / (combination according to claim 1 is comprised between 4 and 3.

8. Preparation of the composition according to any of claims 5 to 7, by mixing into water, polyglyceryl-4 caprate and glyceryl monocaprylate, and optionally a surfactant, a thickening agent and/or a preservative.

9. Use of the composition according to any of claims 5 to 7 as a cosmetic composition.

10. Cleansing composition free of sulfated surfactant comprising a polyglyceryl-4 caprate, a glyceryl monocaprylate and water, wherein the weight ratio polyglyceryl-4 caprate / glyceryl monocaprylate is comprised between 45/55 and 55/45.

11. Method to lower the quantity of sulfated surfactant having cleansing property and optionally foaming property, in a cleansing composition, by adding a combination according to Claim 1 into said cleansing composition.

## Patentansprüche

1. Kombination, umfassend oder bestehend aus Polyglyceryl-4-caprat und Glycerylmonocaprylat, wobei das Gewichtsverhältnis von Polyglyceryl-4-caprat:Glycerylmonocaprylat zwischen 45:55 und 55:45 liegt.

2. Verfahren zur Herstellung einer Kombination durch Mischen eines Polyglyceryl-4-caprats und eines Glycerylmonocaprylats, wobei das Gewichtsverhältnis von Polyglyceryl-4-caprat:Glycerylmonocaprylat zwischen 45:55 und 55:45 liegt.

3. Verwendung der Kombination nach Anspruch 1 als ein Emolliens.

4. Verwendung der Kombination nach Anspruch 1 als ein Reinigungsmittel.

5. Zusammensetzung, umfassend:
- Polyglyceryl-4-caprat;
- Glycerylmonocaprylat; und
- Wasser;
wobei das Gewichtsverhältnis von Polyglyceryl-4-caprat:Glycerylmonocaprylat zwischen 45:55 und 55:45 liegt.

6. Zusammensetzung nach Anspruch 5, weiterhin umfassend ein Tensid, ein Verdickungsmittel und/oder ein Konservierungsmittel.

7. Zusammensetzung nach Anspruch 6, wobei das Tensid ein Tensid mit einer Reinigungseigenschaft und optional einer Schäumeigenschaft ist und wobei das Gewichtsverhältnis (Tenside mit einer Reinigungseigenschaft und optional einer Schäumeigenschaft):(Kombination nach Anspruch 1) zwischen 4 und 3 liegt.

8. Herstellung der Zusammensetzung nach einem der Ansprüche 5 bis 7 durch Mischen von Polyglyceryl-4-caprat und Glycerylmonocaprylat und optional einem Tensid, einem Verdickungsmittel und/oder einem Konservierungsmittel in Wasser.

9. Verwendung der Zusammensetzung nach einem der Ansprüche 5 bis 7 als eine kosmetische Zusammensetzung.

10. Reinigungszusammensetzung, die frei von sulfatiertem Tensid ist, umfassend ein Polyglyceryl-4-caprat, ein Glycerylmonocaprylat und Wasser, wobei das Gewichtsverhältnis von Polyglyceryl-4-caprat:Glycerylmonocaprylat zwischen 45:55 und 55:45 liegt.

11. Verfahren zur Verringerung der Menge von sulfatiertem Tensid mit einer Reinigungseigenschaft und optional einer Schäumeigenschaft in einer Reinigungszusammensetzung durch Zugeben einer Kombination nach Anspruch 1 in die Reinigungszusammensetzung.

## Revendications

1. Combinaison comprenant ou consistant en caprate de polyglycéryle-4 et monocaprylate de glycéryle, dans laquelle le rapport en poids caprate de polyglycéryle-4/monocaprylate de glycéryle est compris entre 45/55 et 55/45.

2. Procédé pour préparer une combinaison par mélange d'un caprate de polyglycéryle-4 et d'un monocaprylate de glycéryle, dans lequel le rapport en poids caprate de polyglycéryle-4/monocaprylate de glycéryle est compris entre 45/55 et 55/45.

3. Utilisation de la combinaison selon la revendication 1 en tant qu'agent émollient.

4. Utilisation de la combinaison selon la revendication 1 en tant qu'agent nettoyant.

5. Composition contenant :
- du caprate de polyglycéryle-4 ;
- du monocaprylate de glycéryle ; et
- de l'eau ;
dans laquelle le rapport en poids caprate de polyglycéryle-4/monocaprylate de glycéryle est compris entre 45/55 et 55/45.

6. Composition selon la revendication 5, comprenant en outre un tensioactif, un agent épaississant et/ou un conservateur.

7. Composition selon la revendication 6, dans laquelle le tensioactif est un tensioactif ayant une propriété nettoyante et éventuellement une propriété moussante, et dans laquelle le rapport en poids (tensioactifs ayant une propriété nettoyante et éventuellement une propriété moussante)/(combinaison selon la revendication 1) est compris entre 4 et 3.

8. Préparation de la composition selon l'une quelconque des revendications 5 à 7, par mélange dans de l'eau de caprate de polyglycéryle-4 et de monocaprylate de glycéryle, et éventuellement d'un tensioactif, d'un agent épaississant et/ou d'un conservateur.

9. Utilisation de la composition selon l'une quelconque des revendications 5 à 7 en tant que composition cosmétique.

10. Composition nettoyante exempte de tensioactif sulfaté comprenant un caprate de polyglycéryle-4, un monocaprylate de glycéryle et de l'eau, dans laquelle le rapport en poids caprate de polyglycéryle-4/monocaprylate de glycéryle est compris entre 45/55 et 55/45.

11. Procédé pour réduire la quantité de tensioactif sulfaté ayant une propriété nettoyante et éventuellement une propriété moussante dans une composition nettoyante, par addition d'une combinaison selon la revendication 1 dans ladite composition nettoyante.
